# EUROPEAN PATENT APPLICATION

(11) **EP 1 279 382 A1**
(43) Date of publication of application: **29.01.2003**
(21) Application number: 01128529.3
(22) Date of filing: 29.11.2001
(51) Int. Cl.: A61F 2/06

(54) **Curved stent**

(30) Priority: 26.07.2001 US 916394
(71) Applicant: JOMED NV, 6235 NS Ulestraten (NL)
(72) Inventor: Gianotti, Marc, 8542 Wiesendangen (CH); Ha, Suk-Woo, 8246 Langwiesen (CH); Seibold, Gerd, 72118 Ammerbuch (DE); Michlitsch, Kenneth J., 8200 Schaffhausen (CH); von Oepen, Randolf, 72074 Tübingen (DE)
(74) Representative: Schmitz, Hans-Werner, Dipl.-Ing.

(57) **Abstract**

The present invention provides a stent comprising a tubular flexible body having a wall with a web structure that is expandable from a contracted delivery configuration to deployed configuration. The web structure comprises a plurality of neighboring, interconnected, web patterns, each web pattern composed of adjoining webs. Each adjoining web comprises a central section interposed between two lateral sections, forming concave or convex configurations. Embodiments of the present invention comprising curvature for tracking tortuous anatomy and reducing localized restoring forces are provided. Methods of using stents in accordance with the present invention are also provided.

## Description

The present invention relates to stents. More particularly, the present invention relates to stents having curvature, and that preferably have web structures configured to expand from contracted delivery configurations to expanded deployed configurations.

Various stent designs are known in the art. These stents form vascular prostheses fabricated from biocompatible materials. Stents are typically used to expand and maintain patency of hollow vessels, such as blood vessels or other body orifices. To this end, the stent is often placed into a hollow vessel of a patient's body in a contracted delivery configuration and is subsequently expanded by suitable means, such as by a balloon catheter or through self-expansion, to a deployed configuration.

A stent often comprises a stent body that is expandable from the contracted to the deployed configuration. A common drawback of such a stent is that the stent decreases in length, or foreshortens, along its longitudinal axis as it expands. Such shortening is undesirable because, in the deployed configuration, the stent may not span the entire area inside a vessel or orifice that requires expansion and/or support. Additionally, when implanted in tortuous anatomy, prior art stents may apply hazardous localized restoring forces to the vessels or orifices.

In view of the foregoing, it is an object to provide a stent that is able to reduce localized restoring forces. The solution of this object is achieved by the features of claim 1.

The stent of the present invention comprises curvature adapted to match the curvature of an implantation site within a patient's body lumen or orifice, for example, adapted to match the curvature of a tortuous blood vessel. Curvature matching is expected to reduce potentially harmful restoring forces that are applied to tortuous anatomy by prior art stents. Such restoring forces may cause local irritation of cells due to force concentration. The forces also may cause vessel kinking, which reduces luminal diameter and blood flow, while increasing blood pressure and turbulence. Curvature may be imparted to the stents by a variety of techniques, such as by heat treating the stents while they are arranged with the desired curvature, or plastically deforming the stents to a curved configuration with secondary apparatus, e.g. a curved balloon.

Methods of using stents in accordance with the present invention are also provided.

The above and other objects and advantages of the present invention will be apparent upon consideration of the following detailed description, taken in conjunction with the accompanying drawings, in which like reference numerals refer to like parts throughout, and in which:
FIG. 1 is a side view of a self-expanding stent of the present invention having a curvature relative to a longitudinal axis of the stent;
FIG. 2 is a side view of the stent of FIG. 1 disposed within a delivery catheter;
FIGS. 3A-3C are side views, partially in section, illustrating a method of deploying the stent of FIG. 1 within tortuous anatomy;
FIG. 4 is a schematic view of an optional intravascular ultrasound image provided for positioning of the stent of FIG. 1; and
FIGS. 5A and 5B are side-views of secondary balloon apparatus for imposing curvature on a balloon-expandable stent of the present invention, shown, respectively, in a collapsed delivery configuration, and in an expanded deployed configuration.

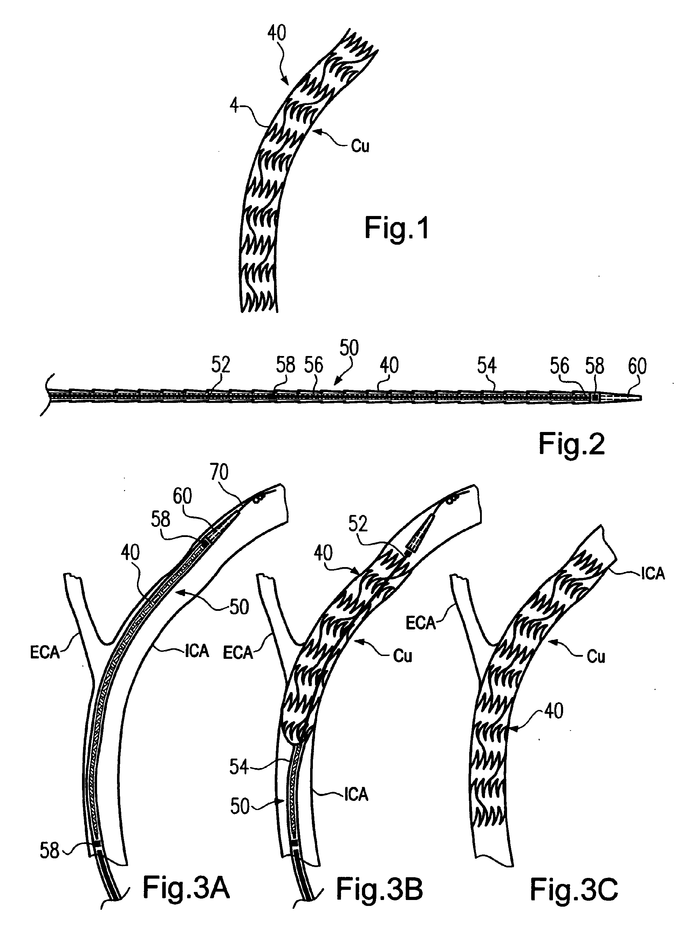

With reference to FIG. 1, an embodiment of stent 1 is described. Prior art stents are commonly formed with substantially straight longitudinal axes. When such a stent is implanted within a tortuous blood vessel, i.e. a blood vessel that does not have a straight longitudinal axis, either the stent or the vessel (or both) deforms to match the profile of the vessel or stent, respectively.

Since previously known self-expanding stents are somewhat flexible, they generally deform at least partially to the curvature of the vessel. However, notably near their ends, these stents also apply localized restoring forces to the wall of the vessel that act to straighten the vessel in the vicinity of the implantation site. As previously known balloon-expandable stents tend to exert higher radial forces, they may apply restoring forces that cause tortuous anatomy to assume the substantially straight profiles of the stents.

For both self-expanding and balloon-expandable embodiments, in circumstances where the vessel wall is thinned or brittle, restoring forces may cause acute puncture or dissection of the vessel, potentially jeopardizing the health of the patient. Alternatively, the restoring forces may cause localized vessel irritation, or may remodel the vessel over time such that it more closely tracks the unstressed, straight profile of the stent. Such remodeling may alter blood flow characteristics through the vessel in unpredictable ways. Restoring forces also may kink the vessel, reducing luminal diameter and blood flow, while increasing blood pressure and turbulence. These and other factors may increase a risk of stenosis or thrombus formation, as well as vessel occlusion.

In FIG. 1, apparatus in accordance with the present invention is provided that is expected to reduce potentially harmful restoring forces applied to tortuous anatomy by prior art stents. Stent 40 comprises curvature **Cu** in an expanded deployed configuration. Stent 40 also illustratively comprises web structure 4 described hereinabove; however, other structures will be apparent to those of skill in the art. The web structure may be formed, for example, by laser-cutting a tubular member, as discussed previously.

Stent 40 comprising curvature **Cu** is preferably self-expanding or balloon-expandable. However, Biflex, wire mesh, and other embodiments will be apparent to those of skill in the art, and fall within the scope of the present invention. Self-expanding embodiments of stent 40 are preferably fabricated from a superelastic material, such as a nickel-titanium alloy, e.g. "Nitinol". Balloon-expandable embodiments may comprise, for example, a stainless steel.

Curvature **Cu** of stent 40 is configured to match the curvature of an implantation site within a patient's body lumen or body orifice, for example, adapted to match the curvature of a tortuous blood vessel. Thus, when implanted within the vessel, neither the vessel nor the stent need deform to match the other's profile. Curvature matching is thereby expected to reduce localized restoring forces at the implantation site. Curvature may be imparted to stent 40 by a variety of techniques, such as by heat treating the stent while it is arranged with the desired curvature, or by plastically deforming the stent with secondary apparatus, e.g. a curved balloon.

Matching of curvature **Cu** with the internal profile of a blood vessel or other body lumen may be accomplished by mapping the internal profile of the body lumen, preferably in 3-dimensional space. Then, curvature **Cu** of stent 40 may be custom-formed accordingly, e.g. by heat treating the stent. Alternatively, secondary apparatus, such as a balloon catheter, may be custom-formed and adapted for plastically deforming stent 40 to impose the curvature. Mapping of the body lumen may be accomplished using a variety of techniques, including ultrasound, e.g. B-mode ultrasound examination, intravascular ultrasound ("IVUS"), angiography, radiography, magnetic resonance imaging ("MRI"), computed tomography ("CT"), and CT angiography.

As an alternative to custom-forming the curvature of stent 40 or the curvature of secondary apparatus for plastically deforming stent 40, a statistical curvature matching technique may be used. Stent 40 or the secondary apparatus may be provided with a standardized curvature **Cu** that more closely matches an average curvature for a desired body lumen within a specific patient population, as compared to prior art stents. As with custom matching, statistical matching of the curvature may be facilitated or augmented by pre-mapping the intended implantation site.

As a further alternative, stent 40 may be manufactured and stocked in a number of different styles, each having its own predetermined curvature. In this manner, a clinician may select a stent having a degree of curvature most appropriate for the specific anatomy presented by the case at hand.

Beneficially, the present invention provides flexibility in providing stents having a wide variety of curvatures/tortuosities, as needed, as will be apparent to those of skill in the art. Stent 40 is expected to have specific utility at tortuous vessel branchings, for example, within the carotid arteries.

Referring now to FIG. 2, a self-expanding embodiment of stent 40, having pre-imposed curvature in the deployed configuration, is shown in a collapsed delivery configuration within delivery catheter 50. Catheter 50 comprises inner sheath 52 having a guide wire lumen, and outer sheath 54 having a lumen sized for disposal about inner sheath 52. Sheath 52 comprises section 56 of reduced cross section. Stent 40 is collapsed about section 56 of inner sheath 52 between optional radiopaque marker bands 58, such that the stent is flush with the remainder of the inner sheath. Marker bands 58 facilitate longitudinal positioning of stent 40 at an implantation site. Outer sheath 54 is disposed over inner sheath 52 and stent 40, in order to maintain the stent in the collapsed delivery configuration. Sheaths 52 and 54 straighten stent 40 while it is in the delivery configuration, thereby facilitating delivery of the stent to an implantation site.

Delivery catheter 50 optionally may comprise imaging transducer 60 that facilitates radial positioning of stent 40, i.e. that facilitates *in vivo* radial alignment of curvature **Cu** of stent 40 with the internal profile of the implantation site. Imaging transducer 60 preferably comprises an IVUS transducer that is coupled to a corresponding imaging system, as described hereinbelow with respect to FIG. 14. An IVUS transducer similar to transducer 60 optionally may also be used to 3-dimensionally map the internal profile of the implantation site prior to advancement of stent 40, thereby allowing custom-manufacture of stent 40.

With reference now to FIGS. 3, a method of using the self-expanding embodiment of stent 40 within tortuous anatomy at a vessel branching is described. In FIGS. 3, stent 40 is illustratively disposed within a patient's carotid arteries, but other implantation sites will be apparent to those of skill in the art. As seen in FIG. 3A, delivery catheter 50, having stent 40 disposed thereon in the collapsed delivery configuration, is advanced over guide wire 70 to an implantation site within internal carotid artery **ICA** that spans the branching of external carotid artery **ECA.** The implantation site may comprise a stenosed or otherwise damaged portion of the artery.

Stent 40 has a curvature **Cu** in the expanded deployed configuration of FIG. 11 that tracks the internal profile of internal carotid artery **ICA** at the implantation site. As discussed previously, curvature Cu may be custom-formed, statistically chosen, or selected from a number of pre-manufactured shapes to better track the curvature of the artery. Such selection may be facilitated or augmented by mapping the profile of the **ICA,** using techniques described hereinabove.

In order to properly align curvature **Cu** of stent 40 with the internal profile of the implantation site within internal carotid artery **ICA,** optional radiopaque marker bands 58 and optional imaging transducer 60 of delivery catheter 50 may respectively be used to longitudinally and radially position stent 40 at the implantation site. Longitudinal positioning of stent 40 may be accomplished by imaging radiopaque marker bands 58, e.g. with a fluoroscope. The implantation site is then positioned between the marker bands, thereby longitudinally orienting stent 40.

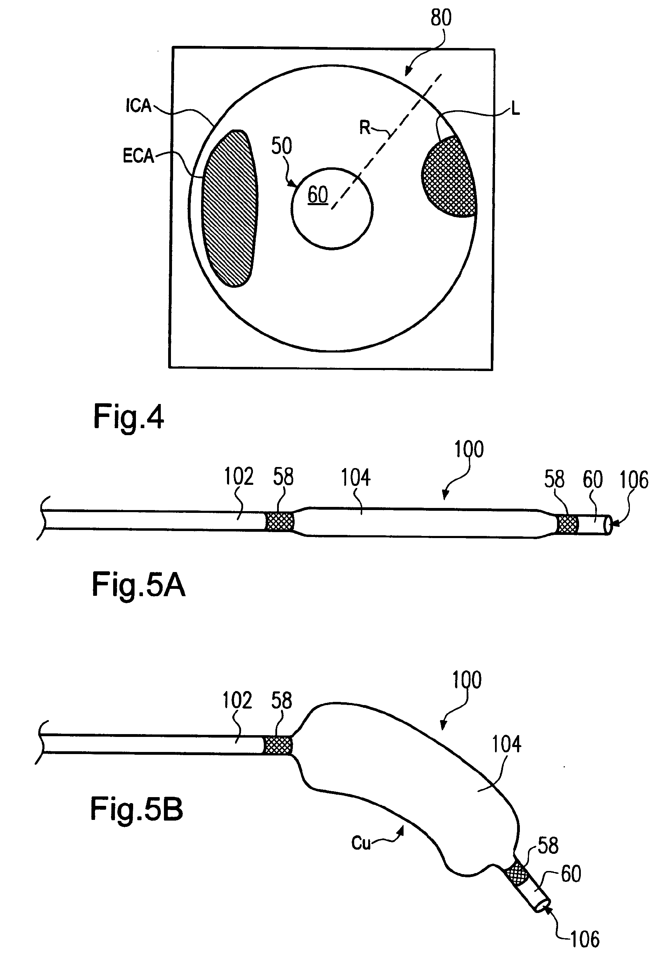

Referring to FIG. 4, in conjunction with FIGS. 3, a technique for radial positioning is described. Imaging transducer 60 preferably comprises an IVUS transducer. Transducer 60 may be either a forward-looking IVUS transducer, or a standard radial-looking IVUS transducer. FIG. 4 provides illustrative IVUS image 80, collected from transducer 60.

In FIG. 4, when using a forward-looking IVUS transducer 60, lumen **L** of internal carotid artery **ICA** can be seen curving away from the longitudinal axis of transducer 60 of delivery catheter 50. Reference line **R** has been superimposed on image 80 and corresponds to the axis of curvature of stent 40. Thus, rotation of catheter 50, and thereby transducer 60 and stent 40, causes rotation of reference line **R** within image 80. In order to radially orient stent 40 with respect to the implantation site, reference line **R** is aligned with lumen **L**.

Referring still to FIG. 4, when using a standard radial-looking IVUS transducer 60, side-branching external carotid artery **ECA** may be imaged. By comparing the position of the external carotid in the IVUS image of FIG. 4 to its position in the fluoroscopic images of FIGS. 3, catheter 50 may be rotated to radially align reference line **R** relative to the position of external carotid artery **ECA** in FIGS. 3, thereby radially aligning curvature **Cu** of stent 40 with the curvature of internal carotid artery **ICA.**

As an alternative technique, both longitudinal and radial positioning of stent 40 may be performed with transducer 60. This is accomplished by creating a 3-dimensional map of the implantation site with transducer 60, by collecting and stacking a series of cross-sectional IVUS images taken along the length of the implantation site. Stent 40 is then positioned with respect to this map. If the vessel was mapped prior to delivery of catheter 50 and stent 40, longitudinal positioning may be accomplished by referencing IVUS image 80 with the previously-conducted mapping, and by advancing catheter 50 until image 80 matches the cross-section of the previous mapping at the proper location.

As yet another technique, both longitudinal and radial positioning of stent 40 may be achieved with radiopaque marker bands 58. Longitudinal positioning may be achieved as described previously, while radial positioning may be achieved by varying the radiopacity of the bands about their circumference, such that the bands comprise a visually recognizable alteration in radiopacity along the axis of curvature of stent 40. This alteration in radiopacity is aligned with the axis of curvature of the implantation site.

Referring back now to FIGS. 3, in FIG. 3B, once stent 40 has been radially and longitudinally oriented with respect to internal carotid artery **ICA,** outer sheath 54 of delivery catheter 50 is gradually withdrawn with respect to inner sheath 52. Stent 40 self-expands to the deployed configuration, and delivery catheter 50 and guide wire 70 are removed from the artery, as in FIG. 3C. Curvature **Cu** of stent 40 tracks the internal profile of internal carotid artery **ICA,** thereby reducing restoring forces applied to the vessel.

With reference to FIGS. 5, secondary apparatus in accordance with the present invention for applying curvature to a balloon-expandable embodiment of stent 40 is described. Secondary apparatus 100 comprises balloon catheter 102 having balloon 104. Secondary apparatus 102 also preferably comprises guide wire lumen 106, as well as radiopaque marker bands 58 and imaging transducer 60, as described hereinabove with respect to FIGS. 3 and 4. Balloon 104, and by extension secondary apparatus 100, is substantially straight in the collapsed delivery configuration of FIG. 5A, but comprises curvature **Cu** in the expanded deployed configuration of FIG. 5B.

Curvature **Cu** may be applied to balloon 104 using techniques described hereinabove. For example, balloon 104 may be heat-treated while the balloon is arranged with the desired curvature. Heat treating of balloon 104 may be accomplished while the balloon is in either the delivery or deployed configuration, or while the balloon is in an intermediary configuration. Additionally, curvature **Cu** of balloon 104 may be matched to the internal profile of a treatment site using, for example, custom-matching or statistical-matching techniques, as described previously.

Embodiments of stent 40 for use with the apparatus of FIGS. 5 are preferably manufactured without curvature **Cu**. As will be clear to those of skill in the art, a balloon-expandable embodiment of stent 40 may be crimped onto balloon 104 while the balloon is in the collapsed delivery configuration. When the balloon is expanded to the deployed configuration at a tortuous treatment site within a patient, curvature **Cu** of balloon 104 plastically deforms stent 40 and imposes curvature **Cu** on the stent. Alignment of curvature **Cu** with the curvature of the tortuous anatomy may be accomplished using, for example, techniques described hereinabove with respect to FIGS. 3 and 4.

Although preferred illustrative embodiments of the present invention are described hereinabove, it will be evident to one skilled in the art that various changes and modifications may be made therein without departing from the invention. For example, stent 40 may further comprise coating **C**, described hereinabove. Additionally, alternative embodiments of secondary apparatus 100 for plastically deforming stent 40, which do not comprise balloons, may be provided. It is intended in the appended claims to cover all such changes and modifications that fall within the true spirit and scope of the invention.

According to a specifically preferred embodiment stent 40 of the present invention may be configured as follows.

The stent may comprise:
a tubular body with a wall having a web structure,
the web structure comprising a plurality of interconnected, neighboring web patterns, each web pattern having a plurality of adjoining webs, each adjoining web comprising a central section interposed between first and second lateral sections,
wherein the central section is substantially parallel to a longitudinal axis of the stent when in the collapsed delivery configuration, each of the first lateral sections joins the central section at a first angle, each of the second lateral sections joins the central section at a second angle, and adjacent ones of the neighboring web patterns have alternating concavity.

The stent may further comprise a first angle that comprises a first obtuse angle, and wherein the second angle comprises a second obtuse angle.

Each adjoining web may have a bowl-like appearance.

The stent may further comprise a plurality of connection elements configured to interconnect the plurality of web patterns.

The stent may further comprise a plurality of transition sections configured to interconnect neighboring web patterns.

The number of adjoining webs may span a circumference of the stent that is selected corresponding to a vessel diameter in which the stent is to be implanted.

The stent may further comprise secondary apparatus for plastically deforming the stent during expansion of the stent from the collapsed delivery configuration to the expanded deployed configuration, thereby imposing the curvature along the longitudinal axis of the stent in the deployed configuration.

The secondary apparatus may comprise a balloon catheter adapted for expansion from a collapsed delivery configuration to an expanded deployed configuration, the balloon catheter comprising curvature along a longitudinal axis of the catheter in the deployed configuration.

The curvature of the balloon catheter may be configured to match an internal profile of an implantation site within a patient's body lumen.

The curvature of the balloon catheter may be configured to match a 3-dimensional map of the internal profile of the implantation site.

The curvature of the balloon catheter may be custom-manufactured or is statistically matched to the internal profile of the implantation site.

The curvature of the balloon catheter may be formed by heat treating the balloon catheter while it is arranged with the desired curvature.

## Claims

1. A stent (40) adapted for expansion from a collapsed delivery configuration to an expanded deployed configuration, the stent (40) having, in the deployed configuration, a curvature (Cu) relative to a longitudinal axis of the stent (40).

2. The stent of claim 1 further comprising a self-expandable structure (4) adapted for expansion from the collapsed delivery configuration to the expanded deployed configuration.

3. The stent of claim 2, wherein the self-expandable structure (4) of the stent (40) is formed by laser-cutting a tubular member.

4. The stent of claim 1, wherein the curvature (Cu) of the stent (40) is configured to match an internal profile of an implantation site within a patient's body lumen.

5. The stent of claim 4, wherein the curvature (Cu) of the stent (40) is configured to reduce restoring forces applied by the stent (40) to the implantation site.

6. The stent of claim 4, wherein the curvature (Cu) of the stent (40) is configured to match a 3-dimensional map of the internal profile of the implantation site.

7. The stent of claim 4, wherein the curvature (Cu) of the stent (40) is custom-manufactured to match the internal profile of the implantation site.

8. The stent of claim 4, wherein the curvature (Cu) of the stent (40) is statistically matched to the internal profile of the implantation site.

9. The stent of claim 1, wherein the curvature (Cu) of the stent is formed by heat treating the stent (40) while it is arranged with the desired curvature.

10. The stent of claim 6, wherein the 3-dimensional map is formed by a technique chosen from the group consisting of ultrasound imaging, intravascular ultrasound imaging, angiography, radiography, magnetic resonance imaging, computed tomography, and computed tomography angiography.

11. The stent of claim 1 further comprising a delivery catheter (50) adapted to selectively maintain the stent (40) in the collapsed delivery configuration.

12. The stent of claim 11, wherein the delivery catheter (50) comprises an inner sheath (52) and an outer sheath (54), the outer sheath (54) removably disposed about the inner sheath (52), the stent (4) concentrically disposed between the inner and outer sheaths (52, 54) in the collapsed delivery configuration.

13. The stent of claim 12, wherein the delivery catheter (50) further comprises radiopaque marker bands (58), the stent (40) disposed between the marker bands (58).

14. The stent of claim 12, wherein the delivery catheter (50) further comprises an imaging transducer (60).

15. The stent of claim 1, wherein the stent (40) is fabricated from a material chosen from the group consisting of superelastic materials, biocompatible materials, and biodegradable materials.

16. The stent of claim 1, wherein the stent (40) is flexible in the collapsed delivery configuration.

17. The stent of claim 1, wherein a thickness of a wall of the stent (40) changes along the longitudinal axis of the stent.

18. The stent of claim 1 further comprising a coating at least partially covering the stent (40).

19. The stent of claim 18 wherein the coating is configured to perform an action chosen from the group consisting of retarding restenosis, retarding thrombus formation, and delivering therapeutic agents to the patient's blood stream.
